# EUROPÄISCHE PATENTSCHRIFT

(11) **EP 1 343 824 B1**
(45) Veröffentlichungstag und Bekanntmachung des Hinweises auf die Patenterteilung: **17.09.2008**
(21) Anmeldenummer: 01990471.3
(22) Anmeldetag: 03.12.2001
(51) Int. Cl.: C07K 17/06, C07K 1/04

(54) **VERFAHREN ZUR IMMOBILISIERUNG VON BIOMOLEKÜLEN, FUNKTIONALISIERTES TRÄGERMATERIAL UND SEINE VERWENDUNG**
METHOD FOR IMMOBILISING BIOMOLECULES, FUNCTIONALISED CARRIER MATERIAL, AND THE USE THEREOF
PROCEDE D'IMMOBILISATION DE BIOMOLECULES, MATERIAU SUPPORT FONCTIONNALISE ET UTILISATION

(30) Priorität: 22.12.2000 DE 10065787
(43) Veröffentlichungstag der Anmeldung: 17.09.2003
(73) Patentinhaber: Poly-An Gesellschaft zur Herstellung von Polymeren für spezielle Anwendungen und Analytik mbH, 13086 Berlin (DE)
(72) Erfinder: WESSIG, Pablo, 13353 Berlin (DE); BENDIG, Jürgen, 10369 Berlin (DE); SCHEDLER, Uwe, 10119 Berlin (DE)
(74) Vertreter: Schneider, Henry
(86) Internationale Anmeldenummer: PCT/EP2001/014072
(87) Internationale Veröffentlichungsnummer: WO 2002/051872

(56) Entgegenhaltungen:
- WO-A-00/17226
- WO-A-89/05616
- WO-A-91/16425
- JAMES I W: "Linkers for Solid Phase Organic Synthesis" TETRAHEDRON, ELSEVIER SCIENCE PUBLISHERS, AMSTERDAM, NL, Bd. 55, Nr. 16, 16. April 1999 (1999-04-16), Seiten 4855-4946, XP004161079 ISSN: 0040-4020

## Beschreibung

Die Erfindung betrifft ein Verfahren zur Immobilisierung von Biomolekülen an einem Trägermaterial, ein mit einem Biomolekül funktionalisiertes Trägermaterial sowie seine Verwendung.

Zur Verfolgung unterschiedlicher Ziele ist es bekannt, Biomoleküle, insbesondere Proteine oder Peptide, aber auch komplexe Moleküle mit Peptidstruktureinheiten und Aminosäuren an Trägermaterialien, der so genannten festen Phase, zu binden. Die Trägermaterialien sind unter den bestimmungsgemäßen, üblicherweise wässrigen Lösungsbedingungen nicht löslich. Ein derartig mit einem Biomolekül funktionalisiertes Trägermaterial wird verwendet, um weitere Biomoleküle, in der Regel ebenfalls Aminosäuren, Peptide, Proteine oder komplexe Moleküle beziehungsweise supramolekulare Systeme mit peptidischen Struktureinheiten, zu fixieren, anzureichern und/oder abzutrennen. Diese Moleküle bilden nach dem Konzept der Antigen-Antikörper-Reaktion spezifisch und strukturselektiv Komplexe mit dem immobilisierten Biomolekül unter Ausbildung nichtkovalenter van-der-Waals-Bindungen und Wasserstoffbrücken. Als Rezeptoreinheiten werden dabei Peptideinheiten und peptidische Sekundärstrukturelemente erkannt (U. Diederichsen, et al., Bioorganic Chemistry, Wiley-Verlag Chemie, Weinheim 1999, S. 221 ff; G. Gauglitz et al., Proc. SPIE 4205: Advanced Environmental and Chemical Sensing Technology, 2000, 10).

Die Immobilisierung des Biomoleküls auf dem Trägermaterial kann durch physikalische Adsorption erfolgen, wobei die beteiligten Bindungskräfte hauptsächlich Wasserstoffbrücken und van-der-Waals-Kräfte sind, oder durch ionische Bindung aufgrund elektrostatischer Kräfte oder durch kovalente Anbindung durch chemische Umsetzung. Die kovalente Immobilisierung von Biomolekülen erfolgt üblicherweise an polymeren Trägermaterialien oder Trägermaterialien, die eine polymere Oberfläche aufweisen. Für die kovalente Anbindung ist es ferner bekannt, das Biomolekül mit leicht aktivierbaren funktionellen Gruppen zu versehen, die in der Lage sind, eine chemische Bindung mit dem Trägermaterial einzugehen. Unter Zuführung der für die chemische Umsetzung erforderlichen Energie durch Erwärmung oder Belichtung mit energiereicher Strahlung werden aus der funktionellen Gruppe des Biomoleküls reaktive Zwischenstufen gebildet, die mit der Polymeroberfläche reagieren und auf dem Wege der kovalenten Anbindung die Aminosäure, das Peptid, das Protein oder das komplexe Molekül mit peptidischer Struktur immobilisieren.

Die WO 91/16425 sowie die US 5,853,744 beschreiben in diesem Zusammenhang den Einsatz azid-derivatisierter Biomoleküle, die bei Erwärmung oder Photolyse unter Stickstoffabspaltung Nitrene bilden, welche insbesondere durch kombinierte Abstraktions- und Insertionsreaktionen mit Polymeroberflächen reagieren und als Amin gemeinsam mit dem Biomolekül immobilisieren. Die WO 91/16425 sowie Clemence et al. (Bioconjugate Chem. 1995, 6, 411-417) beschreiben ferner die Derivatisierung von Biomolekülen mit Diaziridinfunktionen, die nach Aktivierung und nachfolgender Dreiringöffnung sich an die Polymeroberfläche kovalent anbinden. Darüber hinaus offenbart die EP 562373 A die Nutzung von epoxyderivatisierten polymeren Trägern zur kovalenten Anbindung biochemischer Substanzen.

Es ist ferner bekannt, eine Sensibilisatorfähigkeit von Benzophenonstrukturen hinsichtlich der photochemischen H- beziehungsweise Protonenabstraktion (sowohl H⁺ als auch H^{•}) mit anschließender Rekombination der intermediären Radikale zur Immobilisierung von Aminosäuren und Peptiden zu nutzen. Dabei werden die zu immobilisierenden Rezeptoreinheiten (Aminosäure, Peptid) zusätzlich mit einer Benzophenonstruktur versehen. Bei photochemischer Anregung dieses Konjugates in der Nähe einer Polymeroberfläche erfolgt H- beziehungsweise Protonenabstraktion (sowohl H⁺ als auch H^{•}) vom Polymer beziehungsweise vom Peptid durch das angeregte Benzophenon verbunden mit der Bildung von Radikalen seitens des Polymers und/oder des Peptides, die sodann Kombinationsreaktionen eingehen. Im Verlaufe dieser Radikalkombinationen wird die funktionalisierte, an das Polymer anzubindende Rezeptoreinheit zumindest teilweise an der Oberfläche des Polymers kovalent angebunden und damit das Biomolekül immobilisiert (M. Ulbricht et al., J. Membr. Sci. 1996, 120, 239-259; US 5071529; J.-F. Clemence et al., Bioconjugate Chem. 1995, 6, 411-417).

Der Erfindung liegt die Aufgabe zugrunde, ein neues Verfahren zur Immobilisierung von Biomolekülen bereitzustellen, welches sich durch eine hohe Zuverlässigkeit und eine gute Ausbeute auszeichnet, unter schonenden Bedingungen durchführbar ist und hinsichtlich der einsetzbaren Trägermaterialien und Biomoleküle eine hohe Variabilität aufweist. Es soll ferner ein mit einem Biomolekül funktionalisiertes Trägermaterial zur Verfügung gestellt werden, welches mit dem Verfahren herstellbar ist.

Der erste Aspekt dieser Aufgabe wird durch ein Verfahren mit den Merkmalen des Anspruchs 1 gelöst. Das erfindungsgemäße Verfahren zur Immobilisierung von Biomolekülen, insbesondere von Aminosäuren, Peptiden, Proteinen oder Molekülen mit mindestens einer peptidischen Struktureinheit, umfasst die Schritte
(a) Umsetzung des zu immobilisierenden Biomoleküls mit einer Linkerverbindung nach der allgemeinen Formel (I) 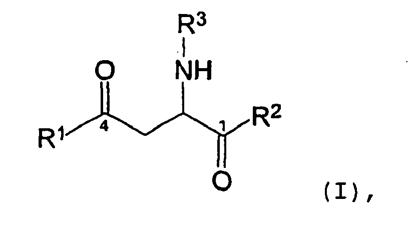 unter Ausbildung einer kovalenten Bindung zwischen dem Biomolekül und der C1-Position oder der Aminogruppe der Linkerverbindung, wobei R² die Bedeutung OR⁴ oder NR⁴R⁵ hat; R¹, R⁴ und R⁵ unabhängig voneinander H, eine Alkylgruppe oder eine Arylgruppe bedeuten; R³ H, eine Alkyl-, eine Aryl-, eine Acyl-, eine Alkoxycarbonyl- oder eine Aryloxycarbonylgruppe bedeutet; und die Alkyl-, Aryl-, Acyl-, Alkoxycarbonyl- und/oder Aryloxycarbonylgruppe der Reste R¹, R³, R⁴ und R⁵ unabhängig voneinander substituiert oder unsubstituiert sind;
(b) Aufbringen des mit der Linkerverbindung verbundenen Biomoleküls auf eine polymere Oberfläche eines Trägermaterials; und
(c) Bestrahlung der Oberfläche mit Licht des UV-vis-Spektralbereiches unter Ausbildung einer kovalenten Bindung zwischen der C4-Position der Linkerverbindung und der polymeren Oberfläche.

Die erfindungsgemäße Linkerverbindung lässt sich in ihrer einfachsten Struktur auf ein Glycin zurückführen, das an seiner. C^{α}-Position mit einer 2-Oxo-ethyl-Einheit, über die letztendlich die kovalente Bindung an das Trägermaterial erfolgt, derivatisiert ist. Abhängig von der Wahl der Reste R² und R³ ergeben sich somit für die Anbindung des zu immobilisierenden Biomoleküls zwei funktionelle Gruppen.

Gemäß einer bevorzugten Ausgestaltung des Verfahrens ist die kovalente Bindung zwischen dem Biomolekül und der Linkerverbindung eine Peptidbindung, die wahlweise entweder zwischen einem N-Terminus des Biomoleküls und der C1-Position der Linkerverbindung und/oder zwischen einem C-Terminus des Biomoleküls und der Aminogruppe der Linkerverbindung gebildet wird. Falls eine selektive N- oder C-terminale Anbindung des Biomoleküls an die Linkerverbindung gewünscht ist, kann diese durch Blockieren des N-Terminus beziehungsweise des C-Terminus des Biomoleküls und/oder der C1-Position beziehungsweise der Aminogruppe der Linkerverbindung mit chemischen Schutzgruppen gesteuert werden. Die Verwendung chemischer Schutzgruppen zur regiospezifischen Reaktionsführung ist dem Fachmann geläufig. So lässt sich beispielsweise seitens des Biomoleküls eine Protektion einer N-terminalen Aminogruppe durch Einbringung einer tert-Butoxycarbonylgruppe (Boc) erreichen. Die Boc-Gruppe kann im Anschluss an die Umsetzung mit der Linkerverbindung durch Hydrolyse von der Aminogruppe wieder entfernt werden. Seitens der Linkerverbindung kann eine Steuerung der Anknüpfung des Biomoleküls durch geeignete Auswahl der Reste R² beziehungsweise R³ erfolgen, wobei ebenfalls die Aminofunktion durch eine Boc-Gruppe geschützt werden kann.

Als Trägermaterial und/oder polymere Oberfläche des Trägermaterials sind organische Polymere besonders geeignet. Hier kommen insbesondere Polypropylen, Polyethylen, Polysulfon, Polyethersulfon, Polystyrol, Polyvinylchlorid, Polyacrylnitril, Zellulose, Amylose, Agarose, Polyamid, Polyimid, Polytetrafluorethylen, Polyvinylidendifluorid, Polyester, Polycarbonat, Polyacrylat, Polyacrylamid oder Derivate von diesen in Frage oder ein Copolymere oder Blends von diesen. Daneben können als Trägermaterial auch anorganische und/oder mineralische Materialien, insbesondere Glas, Silikate, keramische Materialien oder Metall, verwendet werden. Darüber hinaus ist die Verwendung von Kompositen aus mindestens einem anorganischen und/oder mineralischen Material und mindestens einem organischen Polymer denkbar. Im Falle reiner anorganischer und/oder mineralischer Trägermaterialien kann eine Beschichtung mit einem der genannten organischen Polymermaterialien erforderlich sein, um eine Anbindung zu ermöglichen.

Hinsichtlich seiner äußeren Gestalt kann das Trägermaterial in Form einer Membran, eines Films, einer Platte, einer Mikrotiterplatte, eines Reaktionsgefäßes, eines Objektträgers, einer Faser, einer Hohlfaser, eines Vlieses, eines Gewebes, eines Pulvers, eines Granulates oder in Form von Partikeln vorliegen. Dabei kann das Trägermaterial jeweils eine poröse oder nichtporöse Struktur aufweisen. Es ist besonders bevorzugt vorgesehen, dass das Trägermaterial in Form einer Membran mit einer symmetrischen oder asymmetrischen Porenstruktur vorliegt, wobei eine Porengröße im Bereich 1 nm bis 10 µm liegen kann.

Das Aufbringen der Linkerverbindung nach Formel (II) auf das Trägermaterial beziehungsweise seine polymere Oberfläche, erfolgt in Abhängigkeit von der äußeren Form des Trägermaterials durch Tränken, Befeuchten oder Beschichten.

Die Belichtung der Oberfläche kann - obwohl nicht zwingend erforderlich - besonders vorteilhaft in Gegenwart eines Sensibilisators durchgeführt werden. Auf diese Weise lässt sich eine Ausbeute der Photoreaktion erhöhen. Die Bestrahlung erfolgt vorzugsweise mit Licht des Wellenlängenbereiches von 250 bis 500 nm. Dabei hängt die Wahl der verwendeten Wellenlänge oder des Wellenlängenbereiches in erster Linie von dem Rest R¹, der polymeren Oberfläche und dem Vorhandensein und der Art des Sensibilisators ab. Geeignete Lichtquellen sind beispielsweise Laser, UV-Röhren oder Quecksilberdampflampen, wobei gegebenenfalls der Wellenlängenbereich durch Verwendung geeigneter Filter begrenzte werden kann. Die lichtinduzierte Kopplung von Molekülen an polymeren Oberflächen ist beispielsweise aus der WO 91/16425 oder der EP 0 562 373 A2 bekannt und soll hier nicht näher erläutert werden.

Nach erfolgter photochemischer Umsetzung werden nicht umgesetzte Linkerverbindung, Nebenprodukte und gegebenenfalls der Sensibilisator durch Waschen mit Wasser, einem organischen Lösungsmittel oder einem Lösungsmittelgemisch entfernt und das funktionalisierte Trägermaterial getrocknet. Das getrocknete Produkt weist eine sehr gute Stabilität auf und kann bei Raumtemperatur über Wochen und Monate aufbewahrt werden.

Die der Erfindung zugrunde liegende Aufgabe wird ferner durch ein funktionalisiertes Trägermaterial mit den Merkmalen des Anspruchs 19 gelöst. Das funktionalisierte Trägermaterial umfasst eine polymere Oberfläche und mindestens ein an diesem über eine Linkerverbindung kovalent gebundenes Biomolekül gemäß der allgemeinen Formel (IV) oder (V) 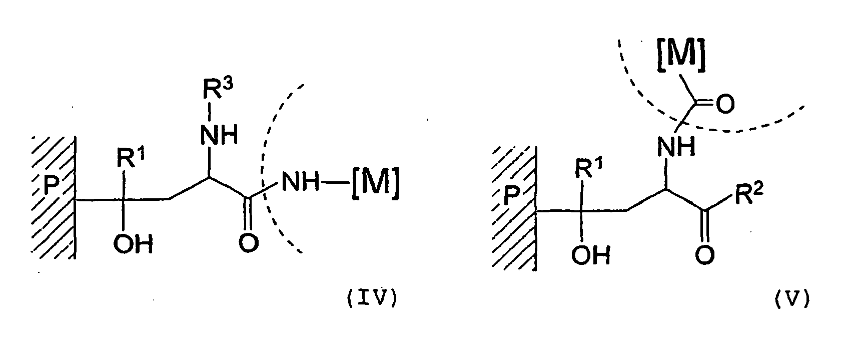 in der P die polymere Oberfläche des Trägermaterials und M das über eine Aminogruppe (Formel IV) oder eine Carbonylgruppe (Formel V) an die Linkerverbindung gebundene Biomolekül bedeutet und R² die Bedeutung OR⁴ oder NR⁴R⁵ hat; R¹, R⁴ und R⁵ unabhängig voneinander H, eine Alkylgruppe oder eine Arylgruppe bedeuten; R³ H, eine Alkyl-, eine Aryl-, eine Acyl-, eine Alkoxycarbonyl- oder eine Aryloxycarbonylgruppe bedeutet; und die Alkyl-, Aryl-, Acyl-, Alkoxycarbonyl- und/oder Aryloxycarbonylgruppe der Reste R¹, R³; R⁴ und R⁵ unabhängig voneinander substituiert oder unsubstituiert sind.

Das funktionalisierte Trägermaterial umfasst demnach ein immobilisiertes Biomolekül, welches über eine gegegebenenfalls substituierte 1-Oxo-2-amino-4-hydroxybutyl-Einheit auf der polymeren Oberfläche des Trägermaterials kovalent gebunden ist. Dabei ist das Biomolekül entweder über seine Aminogruppe an der C1-Carbonylfunktion der Linkerverbindung oder über seine Carbonylgruppe an der Aminofunktion der Linkerverbindung peptidisch gebunden.

Bei dem Biomolekül kann es sich prinzipiell um jedes Molekül handeln, welches über eine für die Anknüpfung geeignete Aminofunktion und/oder eine endständige Carbonyl- beziehungsweise Carboxylfunktion verfügt. Besonders bevorzugt handelt es sich bei dem Biomolekül um eine Aminosäure, ein Peptid, ein Protein oder ein komplexes Molekül mit mindestens einer peptidischen Struktureinheit.

Das erfindungsgemäße funktionalisierte Trägermaterial kann besonders vorteilhaft für die Anreicherung, Albtrennung und/oder Fixierung anderer Aminosäuren, Peptide, Proteine oder komplexerer Moleküle mit Peptidstruktureinheiten verwendet werden.

Weitere bevorzugte Ausgestaltungen der Erfindung ergeben sich aus den übrigen, in den Unteransprüchen genannten Merkmalen.

Die Erfindung wird nachfolgend in Ausführungsbeispielen näher erläutert.

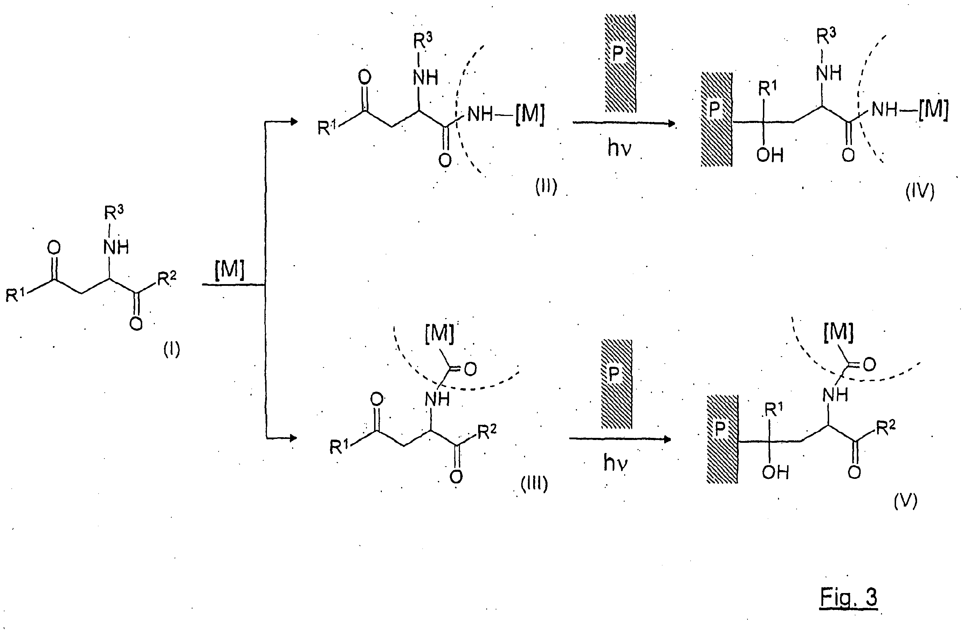

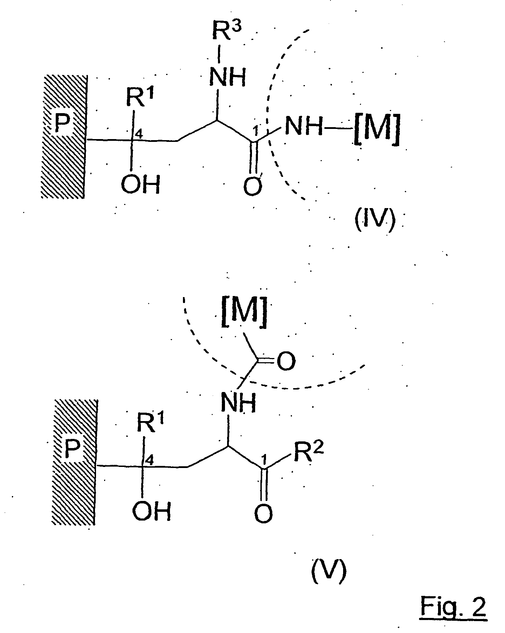

Figur 3 zeigt zunächst eine schematische Übersicht des Immobilisierungsverfahrens gemäß der Erfindung. Die Linkerverbindung gemäß Formel (I) wird in einem ersten Schritt mit einem Biomolekül M, beispielsweise einem Peptid, umgesetzt. Hierfür ergeben sich zwei Anbindungsmöglichkeiten. Erstens kann die Anbindung über eine Aminogruppe des Biomoleküls, etwa den N-Terminus, an die C1-Carbonyl-Position der Linkerverbindung (I) unter Entstehung der Verbindung gemäß der allgemeinen Formel (II) erfolgen. Alternativ oder parallel hierzu kann entsprechend dem unteren Zweig der Figur die Anbindung über eine Carbonyl- beziehungsweise Carboxylgruppe des Biomoleküls, insbesondere dem C-Terminus, an die Aminogruppe der Linkerverbindung erfolgen, woraus sich eine Verbindung gemäß Formel (III) ergibt. Eine regiospezifische Steuerung kann, wie bereits erläutert wurde, durch Verwendung chemischer Schutzgruppen für die jeweils nicht anzubindenden Funktionen der Linkerverbindung und/oder des Biomoleküls erfolgen. In einem anschließenden Schritt wird die Verbindung nach Formel (II) und/oder (III) auf eine polymere Oberfläche P eines Trägermaterials, beispielsweise einer Membran, aufgebracht und mit Licht einer geeigneten Wellenlänge bestrahlt. Unter Erzeugung eines mit dem Biomolekül funktionalisierten Trägermaterials gemäß Formel (IV) und/oder (V) entsteht eine kovalente Bindung zwischen der Linkerverbindung und der Oberfläche.

### Beispiel 1

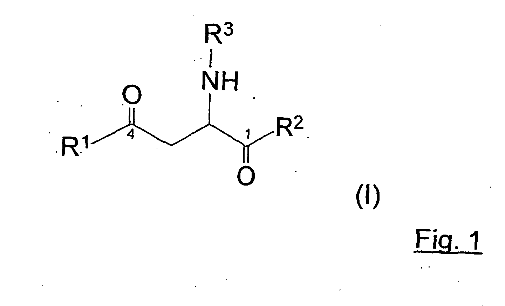

Das zu immobilisierende Peptid H-Ala-Ala-OH wurde als Alanylalanin-methylester-hydrochlorid 2 (7,2 g) mit 10 g L-*N*-Boc-2-(2-oxo-2-phenyl-ethyl)-glycin 1, das heißt mit einer Linkerverbindung der allgemeinen Formel (I) (vergleiche Figuren 1, 3) mit R¹ = Phenyl, R² = OR⁴ R³ = t-Butoxycarbonyl (Boc) und R⁴ - H, und 12 g O-(Benzotriazol-1-yl)-*N,N,N',N*'-tetramethyluronium-tetrafluoroborat in 300 ml Dichlormethan zusammengegeben.

Zu dieser Suspension wurden langsam bei 0°C (Eiskühlung) 1,3 ml Ethyldiisopropylamin unter Rühren zugetropft. Die Suspension wurde auf Raumtemperatur erwärmt und es wurde weiter gerührt, bis die Verbindung **1** nach dünnschichtchromatographischer Detektion (Kieselgel 60 F254, Laufmittel Dichlormethan/Methanol 10:1) nur noch in Spuren nachweisbar war (zirka 2 h). Die Mischung wurde in einen Scheidetrichter gegeben und die organische Phase nacheinander mit je 500 ml Wasser, gesättigter Weinsäurelösung und gesättigter Natriumhydrogencarbonatlösung extrahiert. Die organische Phase wurde abgetrennt, mit Natriumsulfat getrocknet und das Lösungsmittel am Rotationsverdampfer entfernt. Es wurden zirka 15 g eines Harzes erhalten, das durch Flashsäulenchromatographie an Kieselgel 60 (Laufmittel Dichlormethan/Methanol 5:1) gereinigt wurde. Die Ausbeute an 2-[2-(2-tert-Butoxycarbonylamino-4-oxo-4-phenyl-butyrylamino)-propionylaminol-propionsäuremethylester **3** (Boc-BzAla-Ala-Ala-OMe) betrug 13 g.

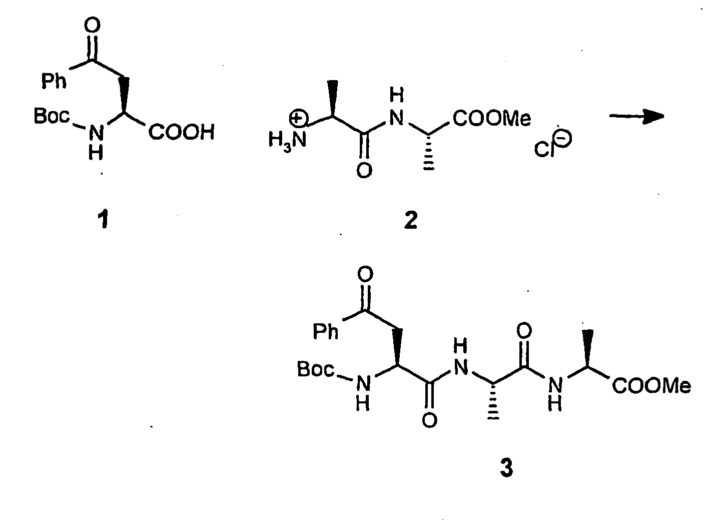

In eine 0,1 M Lösung von 2-[2-(2-tert-Butoxycarbonylamino-4-oxo-4-phenyl-butyrylamino)-propionylamino]-propionsäuremethylester 3, das heißt eine photoreaktive Aminosäure der allgemeinen Formel (II) (vergleiche Figur 3) mit R¹ = Phenyl, und R³ = t-Butoxycarbonyl (Boc), und dem N-terminal als Methylester gebundenen Peptid H-Ala-Ala-OH als Biomolekül M in Dichlormethan wurde eine Polypropylen-Membran (Durchmesser 30 mm) 30 Minuten getaucht und anschließend im Hochvakuum bei 3·10⁻⁵ Torr für 30 Minuten getrocknet.

Die Membran wurde 30 Minuten mit dem Licht einer HBO 500 im Abstand von 20 cm bei Einsatz eines Kantenfilters, der das Licht unterhalb 290 nm herausfiltert, bestrahlt.

Die Membran wurde anschließend fünfmal mit insgesamt 150 ml Dichlormethan gewaschen und 30 Minuten im Hochvakuum bei 3·10⁻⁵ Torr getrocknet.

Der Nachweis der Peptidimmobilisierung entsprechend Formel (IV), in der die Reste R¹ und R³ und das Biomolekül M die oben genannte Bedeutung besitzen, erfolgte durch Vergleich der FT-IR-Spektren für die Polypropylen-Membran vor und nach der Behandlung.

### Beispiel 2

Eine Polypropylen-Membran (Durchmesser 30 mm) wurde in einer Glasschale, gefüllt mit einer 0,1 M Lösung von 2-[2-(2-tert-Butoxycarbonylamino-9-oxo-4-phenyl-butyrylamino)-propionylamino]-propionsäuremethylester **3** (hergestellt nach Beispiel 1), das heißt einer photoreaktive Aminosäure der allgemeinen Formel (II) mit R¹ = Phenyl und R³ = t-Butoxycarbonyl (Boc), und dem N-terminal als Methylester gebundenen Peptid H-Ala-Ala-OH in Benzol, 60 Minuten mit dem Licht einer HBO 500 im Abstand von 20 cm bei Einsatz eines Umlenkspiegels und eines Kantenfilters, der das Licht unterhalb 290 nm herausfiltert, bestrahlt.

Die Membran wurde anschließend einmal mit 50 ml Benzol und zweimal mit 50 ml Dichlormethan gewaschen und 30 Minuten im Hochvakuum bei 3·10⁻⁵ Torr getrocknet.

Der Nachweis der Peptidimmobilisierung entsprechend Formel (IV), in der die Reste R¹ und R³ die oben genannte Bedeutung besitzen, erfolgte durch Vergleich der FT-IR-Spektren für die Polypropylen-Membran vor und nach der Behandlung.

## Patentansprüche

1. Verfahren zur Immobilisierung von Biomolekülen mit den Schritten
(a) Umsetzung des zu immobilisierenden Biomoleküls mit einer Linkerverbindung nach der allgemeinen Formel (I) 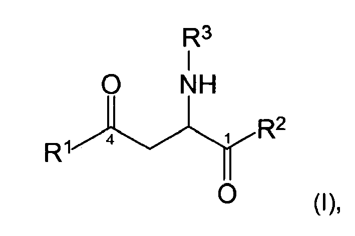 unter Ausbildung einer kovalenten Bindung zwischen dem Biomolekül und der C1-Position oder der Aminogruppe der Linkerverbindung, wobei R² die Bedeutung OR⁴ oder NR⁴R⁵ hat; R¹, R⁴ und R⁵ unabhängig voneinander H, eine Alkylgruppe oder eine Arylgruppe bedeuten; R³ H, eine Alkyl-, eine Aryl-, eine Acyl-, eine Alkoxycarbonyl- oder eine Aryloxycarbonylgruppe bedeutet; und die Alkyl-, Aryl-, Acyl-, Alkoxycarbonyl- und/oder Aryloxycarbonylgruppe der Reste R¹, R³, R⁴ und R⁵ unabhängig voneinander substituiert oder unsubstituiert sind;
(b) Aufbringen des mit der Linkerverbindung verbundenen Biomoleküls auf eine polymere Oberfläche eines Trägermaterials; und
(c) Bestrahlung der Oberfläche mit Licht des UV-vis-Spektralbereiches unter Ausbildung einer kovalenten Bindung zwischen der C4-Position der Linkerverbindung und der polymeren Oberfläche.

2. Verfahren nach Anspruch 1, **dadurch gekennzeichnet, dass** das Biomolekül eine Aminosäure, ein Peptid, ein Protein oder ein Molekül mit mindestens einer peptidischen Struktureinheit ist.

3. Verfahren nach Anspruch 1 oder 2, **dadurch gekennzeichnet, dass** die kovalente Bindung zwischen dem Biomolekül und der Linkerverbindung eine Peptidbindung ist.

4. Verfahren nach Anspruch 3, **dadurch gekennzeichnet, dass** die Peptidbindung wahlweise entweder zwischen einem N-Terminus des Biomoleküls und der C1-Position der Linkerverbindung und/oder zwischen einem C-Terminus des Biomoleküls und der Aminogruppe der Linkerverbindung gebildet wird.

5. Verfahren nach Anspruch 4, **dadurch gekennzeichnet, dass** eine Steuerung der N- oder C-terminalen Anbindung des Biomoleküls an die Linkerverbindung durch Blockieren des N-Terminus oder des C-Terminus des Biomoleküls und/oder der C1-Position oder der Aminogruppe der Linkerverbindung mit chemischen Schutzgruppen erfolgt.

6. Verfahren nach einem der vorhergehenden Ansprüche, **dadurch gekennzeichnet, dass** als Trägermaterial und/oder polymere Oberfläche (P) ein organisches Polymer verwendet wird.

7. Verfahren nach Anspruch 6, **dadurch gekennzeichnet, dass** das organische Polymer Polypropylen, Polyethylen, Polysulfon, Polyethersulfon, Polystyrol, Polyvinylchlorid, Polyacrylnitril, Zellulose, Amylose, Agarose, Polyamid, Polyimid, Polytetrafluorethylen, Polyvinylidendifluorid, Polyester, Polycarbonat, Polyacrylat, Polyacrylamid oder ein Derivat hiervon ist oder ein Copolymer oder ein Blend von diesen.

8. Verfahren nach einem der vorhergehenden Ansprüche, **dadurch gekennzeichnet, dass** als Trägermaterial ein anorganisches und/oder mineralisches Material verwendet wird.

9. Verfahren nach Anspruch 8, **dadurch gekennzeichnet, dass** als Trägermaterial ein Glas, ein Silikat, ein keramisches Material oder ein Metall verwendet wird.

10. Verfahren nach einem der Ansprüche 6 bis 9, **dadurch gekennzeichnet, dass** als Trägermaterial ein Komposit aus mindestens einem anorganischen und/oder mineralischen Material und mindestens einem organischen Polymer verwendet wird.

11. Verfahren nach einem der vorhergehenden Ansprüche, **dadurch gekennzeichnet, dass** das Trägermaterial in Form einer Membran, eines Films, einer Platte, einer Mikrotiterplatte, eines Reaktionsgefäßes, eines Objektträgers, einer Faser, einer Hohlfaser, eines Vlieses, eines Gewebes, eines Pulvers, eines Granulates, oder von Partikeln eingesetzt wird und jeweils porös oder nichtporös sein kann.

12. Verfahren nach Anspruch 11, **dadurch gekennzeichnet, dass** das Trägermaterial in Form einer Membran mit einer symmetrischen oder asymmetrischen Porenstruktur eingesetzt wird.

13. Verfahren nach Anspruch 12, **dadurch gekennzeichnet, dass** die Porengröße 1 nm bis 10 µm beträgt.

14. Verfahren nach einem der vorhergehenden Ansprüche, **dadurch gekennzeichnet, dass** die Bestrahlung der Oberfläche in Gegenwart oder in Abwesenheit eines Sensibilisators erfolgt.

15. Verfahren nach einem der vorhergehenden Ansprüche, **dadurch gekennzeichnet, dass** die Bestrahlung mit Licht eines Wellenlängenbereiches von 250 bis 500 nm erfolgt.

16. Verfahren nach Anspruch 15, **dadurch gekennzeichnet, dass** als Lichtquelle Laser, UV-Röhren oder Quecksilberdampflampen gegebenenfalls mit geeigneten Filtern eingesetzt werden.

17. Verfahren nach einem der vorhergehenden Ansprüche, **dadurch gekennzeichnet, dass** nach der Bestrahlung nicht umgesetzte Substanzen durch Waschen mit einer Waschflüssigkeit entfernt werden.

18. Verfahren nach Anspruch 17, **dadurch gekennzeichnet, dass** als Waschflüssigkeit Wasser, ein organisches Lösungsmittel oder ein Lösungsmittelgemisch verwendet wird.

19. Funktionalisiertes Trägermaterial gemäß der allgemeinen Formel (IV) oder (V) mit einer polymeren Oberfläche und mindestens einem an diesem über eine Linkerverbindung kovalent gebundenen Biomolekül 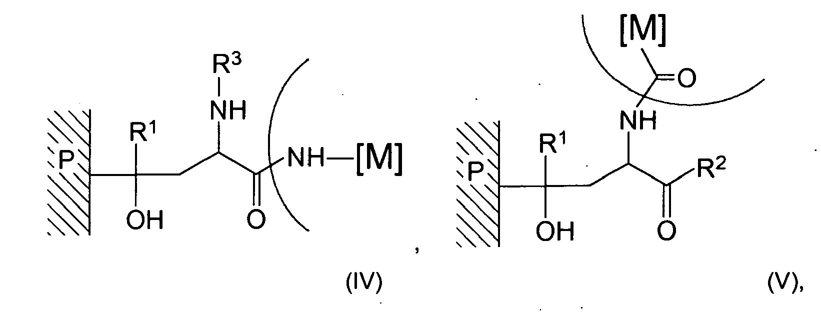 in der P die polymere Oberfläche des Trägermaterials und M das über eine Aminogruppe (Formel IV) oder eine Carbonylgruppe (Formel V) an die Linkerverbindung gebundene. Biomolekül bedeutet und R² die Bedeutung OR⁴ oder NR⁴R⁵ hat; R¹, R⁴ und R⁵ unabhängig voneinander H, eine Alkylgruppe oder eine Arylgruppe bedeuten; R³ H, eine Alkyl-, eine Aryl-, eine Acyl-, eine Alkoxycarbonyl- oder eine Aryloxycarbonylgruppe bedeutet; und die Alkyl-, Aryl-, Acyl-, Alkoxycarbonyl- und/oder Aryloxycarbonylgruppe der Reste R¹, R³, R⁴ und R⁵ unabhängig voneinander substituiert oder unsubstituiert sind.

20. Funktionalisiertes Trägermaterial nach Anspruch 19, **dadurch gekennzeichnet, dass** das Biomolekül (M) eine Aminosäure, ein Peptid, ein Protein oder ein Molekül mit mindestens einer peptidischen Struktureinheit ist.

21. Funktionalisiertes Trägermaterial nach einem der Ansprüche 19 oder 20, **dadurch gekennzeichnet, dass** das funktionalisierte Trägermaterial nach einem der Ansprüche 1 bis 18 herstellbar ist.

22. Verwendung eines funktionalisierten Trägermaterials nach einem der Ansprüche 19 bis 21 zur Anreicherung, Abtrennung und/oder Fixierung von Aminosäuren, Peptiden, Proteinen und/oder Molekülen mit mindestens einer Peptidstruktureinheit.

## Claims

1. Method for the immobilisation of bio-molecules comprising the steps
(a) transformation of the bio-molecule which is to be immobilised with a linker compound according to the general formula (I) 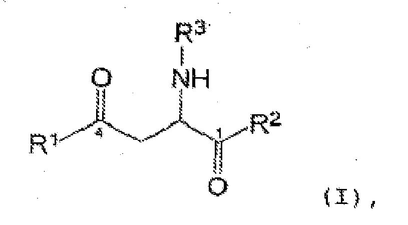 with the formation of a co-valent linkage between the bio-molecule and the C1 position or the amino group of the linker compound, with R² having the meaning OR⁴ or NR⁴R⁵; R¹, R⁴ and R⁵ independent of each other meaning H, an alkyl group or an aryl group; R³ meaning H, an alkyl, an aryl, an acyl, an alkoxycarbonyl or an aryloxycarbonyl group; and the alkyl, aryl, acyl, alkoxycarbonyl and/or aryloxycarbonyl group of the residues R¹, R³, R⁴ and R⁵ being substituted or unsubstituted independent of each other;
(b) application of the bio-molecule connected with the linker compound on a polymeric surface of a carrier material; and
(c) radiation of the surface with light of the UV-vis spectral range with the formation of a co-valent binding between the C4 position of the linker compound and the polymeric surface.

2. Method according to Claim 1 **characterised in that** the bio-molecule is an amino acid, a peptide, a protein or a molecule with at least one peptidic structural unit.

3. Method according to Claim 1 or Claim 2 **characterised in that** the co-valent binding between the bio-molecule and the linker compound is a peptide binding.

4. Method according to Claim 3 **characterised in that** the peptide binding is optionally formed either between a N terminus of the bio-molecule and the C1 position of the linker compound and/or between a C terminus of the bio-molecule and the amino group of the linker compound.

5. Method according to Claim 4 **characterised in that** a control of the N or C terminus binding of the bio-molecule to the linker compound is made by means of blocking the N terminus or the C terminus of the bio-molecule and/or of the C1 position or of the amino group of the linker compound with chemical protective groups.

6. Method according to any of the preceding claims **characterised in that** an organic polymer is used as carrier material and/or as polymeric surface (P).

7. Method according to Claim 6 **characterised in that** the organic polymer is polypropylene, polyethylene, polysulfone, polyethersulfone, polystyrene, polyvinylchloride, polyacrylonitrile, cellulose, amylose, agarose, poylamide, polyimide, polytetrafluoroethylene, polyvinylidenedifluoride, polyester, polycarbonate, polyacrylate, polyacrylamide or a derivative thereof or a co-polymer or a blend thereof.

8. Method according to any of the preceding claims **characterised in that** an inorganic and/or mineral material is used as carrier material.

9. Method according to Claim 8 **characterised in that** a glass, a silicate, a ceramic material or a metal is used as carrier material.

10. Method according to any of the Claims 6 to 9 **characterised in that** a composite of at least one inorganic and/or mineral material and at least one organic polymer is used as carrier material.

11. Method according to any of the preceding claims **characterised in that** the carrier material is used in the form of a membrane, a film, a plate, a microtiter plate, a reaction vessel, a specimen holder, a fibre, a hollow fibre, a non-woven fabric, a fabric, a powder, a granulate or particles and may in each case be either porous or non-porous.

12. Method according to Claim 11 **characterised in that** the carrier material is used in the form of a membrane with a symmetric or asymmetric pore structure.

13. Method according to Claim 12 **characterised in that** the pore size is 1 nm to 10 µm

14. Method according to any of the preceding claims **characterised in that** the radiation of the surface is made in the presence or in the absence of a sensitiser.

15. Method according to any of the preceding claims **characterised in that** the radiation is made with light of a wavelength range from 250 to 500 nm.

16. Method according to Claim 15 **characterised in that** laser, UV tubes or mercury vapour lamps, if appropriate with suitable filters, are used as light source.

17. Method according to any of the preceding claims **characterised in that** after the radiation substances which have not been transformed are removed by washing with a washing liquid.

18. Method according to Claim 17 **characterised in that** water, an organic solvent or a mixture of solvents is used as washing liquid.

19. Functionalised carrier material according to the general formula (IV) or (V) with a polymeric surface and at least one bio-molecule co-valently bound to the same via a linker compound 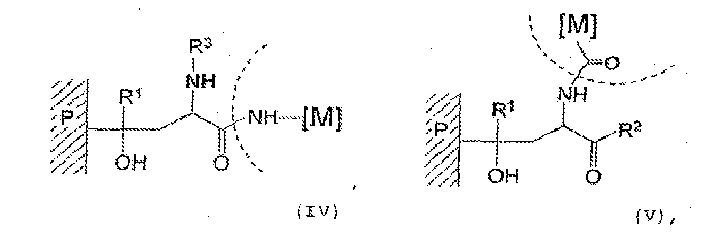 where P means the polymeric surface of the carrier material and M means the bio-molecule bound to the linker compound via an amino group (formula IV) or a carbonyl group (formula V) and R² has the meaning OR⁴ or NR⁴R⁵; R¹, R⁴ and R⁵ independent of each other meaning H, an alkyl group or an aryl group; R³ meaning H, an alkyl, an aryl, an acyl, an alkoxycarbonyl or an aryloxycarbonyl group; and the alkyl, aryl, acyl, alkoxycarbonyl and/or aryloxycarbonyl group of the residues R¹, R³, R⁴ and R⁵ being substituted or unsubstituted independent of each other.

20. Functionalised carrier material according to Claim 19 **characterised in that** the bio-molecule (M) is an amino acid, a peptide, a protein or a molecule with at least one peptidic structural unit.

21. Functionalised carrier material according to any of the Claims 19 or 20 **characterised in that** the functionalised carrier material can be produced in accordance with any of the Claims 1 to 18.

22. Use of a functionalised carrier material according to any of the Claims 19 to 21 for enriching, separating and/or fixing amino acids, peptides, proteins and/or molecules with at least one peptidic structural unit.

## Revendications

1. Procédé d'immobilisation de biomolécules, comportant les étapes
(a) Conversion de la biomolécule à immobiliser avec une combinaison de linker selon la formule générale (I) 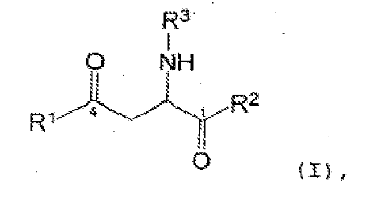 avec formation d'une liaison covalente entre la biomolécule et la position C1 ou le groupement amino de la combinaison de linker, R² représentant OR⁴ ou NR⁴R⁵ ; R¹, R⁴ et R⁵ représentant, indépendamment les uns des autres, H, un groupement alkyle ou un groupement aryle ; R³ représentant H, un groupement alkyle, aryle, acyle, alkoxycarbonyle ou un groupement aryloxycarbonyle ; et les groupements alkyle, aryle, acyle, alkoxycarbonyle et/ou aryloxycarbonyle des restes R¹, R³, R⁴ et R⁵ étant substitués ou non substitués indépendamment les uns des autres ;
(b) Mise en place de la biomolécule liée avec la combinaison de linker sur une surface polymère d'un matériau support ; et
(c) irradiation de la surface avec une lumière de la plage spectrale UV-vis avec formation d'une liaison covalente entre la position C4 de la combinaison de linker et la surface polymère.

2. Procédé selon la revendication 1, **caractérisé en ce que** la biomolécule est un acide aminé, un peptide, une protéine ou une molécule avec au moins une unité structurelle peptidique.

3. Procédé selon la revendication 1 ou 2, **caractérisé en ce que** la liaison covalente entre la biomolécule et la combinaison de linker est une liaison peptide.

4. Procédé selon la revendication 3, **caractérisé en ce que** la liaison peptide est formée au choix soit entre un N-terminus de la biomolécule et la position C1 de la combinaison de linker et/ou soit entre un C-terminus de la biomolécule et le groupement amino de la combinaison de linker.

5. Procédé selon la revendication 4, **caractérisé en ce qu'**une commande de la liaison N-terminale ou C-terminale de la biomolécule avec la combinaison de linker s'effectue par blocage du N-terminus ou du C-terminus de la biomolécule et/ou de la position C1 ou du groupement amino de la combinaison de linker avec des groupements de protection chimiques.

6. Procédé selon une des revendications précédentes, **caractérisé en ce que**, comme matériau support et/ou surface polymère (P), un polymère organique est utilisé.

7. Procédé selon la revendication 6, **caractérisé en ce que** le polymère organique est polypropylène, polyéthylène, polysulfone, polyéthersulfone, polystyrène, polychlorure de vinyle, polyacrylonitrile, cellusose, amylose, agarose, polyamide, polyimide, polytétrafluoréthylène, polydifluorure de vinylidène, polyester, polycarbonate, polyacrylate, polyacrylamide ou un déviré de ceux-ci, ou un copolymère ou un mélange de ceux-ci.

8. Procédé selon une des revendications précédentes, **caractérisé en ce que**, comme matériau support, un matériau inorganique et/ou minéral est utilisé.

9. Procédé selon la revendication 8, **caractérisé en ce que**, comme matériau support, un verre, un silicate, un matériau céramique ou un métal est utilisé.

10. Procédé selon une des revendications 6 à 9, **caractérisé en ce que**, comme matériau support, un composite d'au moins un matériau inorganique et/ou minéral et d'au moins un polymère organique est utilisé.

11. Procédé selon une des revendications précédentes, **caractérisé en ce que** le matériau support est mis en oeuvre sous la forme d'une membrane, d'un film, d'une plaque, d'une microplaque de titrage, d'un réacteur, d'une lame porte-objets, d'une fibre, d'une fibre creuse, d'un voile, d'un tissu, d'une poudre, d'un granulat ou de particules, et peut être respectivement poreux ou non poreux.

12. Procédé selon la revendication 11, **caractérisé en ce que** le matériau support est mis en oeuvre sous la forme d'une membrane ayant une structure de pores symétrique ou asymétrique.

13. Procédé selon la revendication 12, **caractérisé en ce que** la taille des pores est de 1 nm à 10 µm.

14. Procédé selon une des revendications précédentes, **caractérisé en ce que** l'irradiation de la surface s'effectue en présence ou en absence d'un sensibilisateur.

15. Procédé selon une des revendications précédentes, **caractérisé en ce que** l'irradiation s'effectue avec une lumière d'une plage de longueur d'onde de 250 à 500 nm.

16. Procédé selon la revendication 15, **caractérisé en ce que**, comme source lumineuse, un laser, des tubes UV ou des lampes à vapeur de mercure éventuellement avec des filtres appropriés sont utilisés.

17. Procédé selon une des revendications précédentes, **caractérisé en ce que**, après l'irradiation, les substances non converties sont enlevées par lavage avec un liquide de lavage.

18. Procédé selon la revendication 17, **caractérisé en ce que**, comme liquide de lavage, de l'eau, un solvant organique ou un mélange de solvant est utilisé(e).

19. Matériau support fonctionnalisé selon la formule générale (IV) ou (V), avec une surface polymère et au moins une biomolécule liée de façon covalente à ce matériau par le biais d'une combinaison de linker 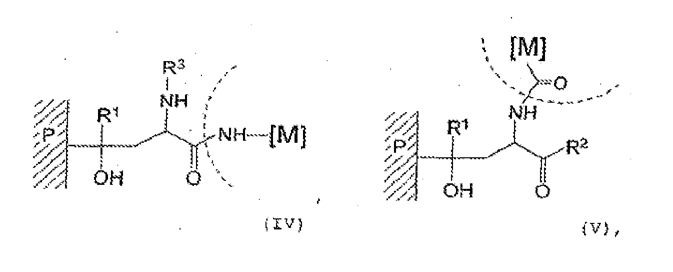 dans laquelle P représente la surface polymère du matériau support et M la biomolécule liée, par le biais d'un groupement amino (formule IV) ou d'un groupement carbonyle (formule V), à la combinaison de linker, et R² représente OR⁴ ou NR⁴R⁵ ; R¹, R⁴ et R⁵ représentent, indépendamment les uns des autres, H, un groupement alkyle ou un groupement aryle ; R³ représentant H, un groupement alkyle, aryle, acyle, alkoxycarbonyle ou un groupement aryloxycarbonyle ; et le groupement alkyle, aryle, acyle, alkoxycarbonyle et/ou aryloxycarbonyle des restes R¹, R³, R⁴ et R⁵ sont substitués ou non substitués indépendamment les uns des autres.

20. Matériau support fonctionnalisé selon la revendication 19, **caractérisé en ce que** la biomolécule (M) est un acide aminé, un peptide, une protéine ou une molécule ayant au moins une unité structurelle peptidique.

21. Matériau support fonctionnalisé selon une des revendications 19 ou 20, **caractérisé en ce que** le matériau support fonctionnalisé peut être réalisé selon une des revendications 1 à 18.

22. Utilisation d'un matériau support fonctionnalisé selon une des revendications 19 à 21 pour l'enrichissement, la séparation et/ou la fixation d'acides aminés, de peptides, de protéines et/ou de molécules ayant au moins une unité structurelle de peptides.
